# EUROPEAN PATENT APPLICATION

(11) **EP 4 119 152 A1**
(43) Date of publication of application: **18.01.2023**
(21) Application number: 21767693.1
(22) Date of filing: 03.03.2021
(51) Int. Cl.: A61K 38/00, A61P 35/00

(54) **APPLICATION OF IFN-y IN PREPARING ANTI-TUMOR ADJUVANT DRUG**

(30) Priority: 09.03.2020 CN 202010159630; 01.06.2020 CN 202010487110
(71) Applicant: West China Hospital of Sichuan University, Chengdu, Sichuan 610041 (CN)
(72) Inventor: YANG, Hanshuo, Chengdu, Sichuan 610041 (CN); DONG, E, Chengdu, Sichuan 610041 (CN); YUE, Xiaozhu, Chengdu, Sichuan 610041 (CN)
(74) Representative: Dolphin, Kirsty Mairi
(86) International application number: PCT/CN2021/078858
(87) International publication number: WO 2021/179967

(57) **Abstract**

The present invention provides a use of IFN-γ in preparing an anti-tumor adjuvant drug, wherein the IFN-γ enhances killing effect of the T cell preparation on tumor cell by sensitizing the tumor cell; the T cell preparation comprises non-genetically engineered T cell and/or genetically engineered T cell; and the IFN-γ comprises full-length or fragment of wild-type or mutant IFN-γ. The present invention revises the current understanding of IFN-γ in the prior art and finds that the IFN-γ inhibits the acquired immune resistance mediated by PD-L1-PD-1 and enhances the anti-tumor effect of immunotherapy by activating the IFN-γ signaling pathway in tumor cell.

## Description

### Priority Application

. The present application claims priority from Chinese invention patent application CN2020101596309 "APPLICATION OF IFN-γ IN PREPARING ANTI-TUMOR ADJUVANT DRUG" filed on March 9, 2020, and Chinese invention patent application CN2020104871100"A CAR EXPRESSION VECTOR AND APPLICATION THEREOF" filed on June 1, 2020, which are incorporated by reference in their entirety.

### Technical Field

. The present invention belongs to the field of biomedicine, and particularly relates to an anti-tumor drug for assisting anti-tumor immunotherapy and an application thereof.

### Background

. Tumor immunotherapy is a revolutionary progress in anti-tumor therapy, which can fight tumors by mobilizing a body's own immune function. The CAR-T therapy belongs to the field of immunotherapy. CAR-T cells have achieved surprising effect in the treatment of hematologic tumors by targeting CD19. By April 2018, there are a total of 2,513 immunotherapies for the tumors, 382 in China, and 227 in clinical phases III and IV. Kymriah (Novartis company) and Yescarta (Kite company) that have been on the market are respectively used for treating childhood acute lymphoblastic leukemia and adult B-cell lymphoma. The complete response (CR) of Kymriah is 82.5%, and the relapse probability is 75% by 6 months and is 64% by 12 months. For another FDA-approved CD19 specific CAR-T product, Yescarta, can achieve 43 to 52% of CR in treating patients with relapsed or refractory DLBCL.

. However, for solid tumors, the therapeutic effect of CAR-T is not good. For example, in phase I clinical studies of EGFR-CAR-T in the treatment of epidermal growth factor receptor cells in relapsed/refractory EGFR + non-small cell lung cancer, there were only 2 respondents and 5 patients with stable disease (SD) among 11 cancer patients. The feasibility and safety of administering HER2-CAR-T cells to patients with relapsed or refractory HER2-positive tumors were evaluated in phase I/II studies of a dose-escalation clinical trial. However, the clinical benefits were limited, with only 4/17 evaluable patients showing SD. That is, the therapeutic effect of CAR-T cells in solid tumors is limited. And the reasons for the poor efficacy of CAR-T cells on solid tumors are not very clear at present, but it is speculated that there may be various reasons, such as poor survival of CAR-T cells in vivo which affects their killing efficacy due to their short-lifespan after input into the body; and solid tumors can form an immunosuppressive microenvironment due to pluripotency in metabolism, immune escape, and tissue formation, which makes it difficult for CAR-T cells to infiltrate into solid tumor tissue to exert their efficacy. Even when the CAR-T cells enter the solid tumors, the CAR-T cells will be inactivated and die due to the restriction of immune system in many aspects. Therefore, how to enhance and promote the therapeutic effect of CAR-T cells on solid tumors is an urgent problem to be solved in the prior art.

. As an inhibitory molecule in costimulatory signals, the main function of PD-1 was first discovered to induce apoptosis of T cell, thus preventing the immune system from killing cancer cells. In addition, PD-1 can negatively regulate T cell-mediated immune responses by binding to its ligand, programmed death ligand 1 (PD-L1), that is, the tumor cells "deceive" T cells and escape recognition by T cells via the binding of PD-L1 to PD-1. Therefore, blocking this signaling pathway is considered as an effective cancer immunotherapy. Since the approval of the first PD-1 inhibitors, pembrolizumab and nivolumab, in 2014, there has been unprecedented growth in the clinical development of PD-1/PD-L1 inhibitors as a form of cancer immunotherapy. PD-1 and PD-L1 inhibitors in clinical trials can be used for various types of cancer, comprising advanced melanoma, non-small cell lung cancer, renal cell carcinoma, bladder cancer, hodgkin lymphoma, liver cancer, head and neck squamous cell carcinoma, urothelial carcinoma, Merkel cell carcinoma, etc. So far, there are three PD-1 inhibitors that have been on the market abroad: Pembrolizumab, Nivolumab and Cemiplimab; and there are four inhibitors that have been on the market in China: Toripalimab, Tislelizumab, Tyvyt and Camrelizumab. The PD-L1 inhibitors that have been on the market are respectively: Atezolizumab, Avelumab and Durvalumab. When PD-L1/PD-1 therapy is applied to more solid tumors, how to make the PD-L1/PD-1 therapy achieve better anti-tumor therapeutic effect is a problem to be solved at present.

. An adoptive cell therapy (ACT) is an effective anti-cancer strategy, known as "living drug", which plays an important role in cancer immunotherapy. ACT involves the isolation, expansion in vitro and functional identification of immunocompetent cells from tumor patients, and then transfer back to patients to directly kill the tumors or stimulate the body's immune response to kill the tumors. At present, ACT mainly comprises TIL, LAK, CIK, DC, NK, TCR-T and CAR-T.

. In order to enhance the therapeutic effect of CAR-T cells, the current strategies mainly focus on further genetic engineering or nanomodification for CAR-T cells. For example, the insertion of multi-target CAR genes into CAR-T cells which effectively affects the expansion and persistence of T cells by optimizing the structure of CAR; or enabling CAR-T cells to express chemokines or direct modification of adhesion molecules or chemical groups to enhance the orientation and recognition of CAR-T cells to tumor tissue. However, there are some problems and inadequacies in these technical means more or less. Taking multi-target CAR-T cells as an example, excessive insertion of foreign genes can affect the original activity of the T cells. Therefore, the modification to the CAR-T cells must comprehensively consider multiple positive and negative factors. It is necessary to compare and balance the positive and negative effects brought by related modifications. Finally, more targeted and more effective modification methods are selected.

. Studies show that PD-1, as an inhibitory signaling pathway, is an important factor affecting the therapeutic effect of CAR-T. Blocking the PD-1 pathway can improve the anti-tumor effect of CAR-T cells. Therefore, the technical means for inhibiting the PD-1 is considered as a feasible CAR-T modification direction.

. In the existing technical route, an attempt to make CAR-T cells secrete PD-1 or PD-L1 antibodies by genetic engineering can significantly improve the therapeutic effect on solid tumors, or knocking out the gene encoding the PD-1 in CAR-T cells by gene editing can also improve the anti-tumor activity of CAR-T.

. However, all of above these technical solutions and strategies of technical routes focus on improving the functions of the CAR-T cells per se, which have little effect on tumor cells and an immunosuppressive microenvironment in the solid tumors. In other words, current technical solutions and technical routes focus only on improving CAR-T cells per se, but do not further enhance the anti-tumor effect of CAR-T cell therapy from the perspective of acting on tumor cells.

. To sum up, how to improve the anti-tumor effect of strategies in the prior art such as immunotherapy and/or immune checkpoint inhibitor therapy and/or adoptive cellular therapy and propose a more optimized and more effective anti-tumor therapeutic solution are very important problems in the field of tumor immunotherapy at present, and are also technical problems that the present invention tries to address. In addition, the present invention also aims to influence tumor cells and an immunosuppressive microenvironment in solid tumors, and provides an optimized CAR-T cell therapy strategy.

### Summary

. In view of the above technical problems, the present invention focuses on a type II interferon (IFN-γ), provides an anti-tumor adjuvant drug prepared thereof and other applications, and comprises two sub-inventions (namely, A invention and B invention) and respective extensions based on these two inventions.

. One aspect of the present invention provides a use of IFN-γ in preparation of an anti-tumor adjuvant drug; a kit used for detecting anti-tumor effect of an immune checkpoint inhibitor; an anti-tumor drug for assisting anti-tumor immunotherapy; a use of combination of IFN-γ with T cell preparation in preparation of an tumor immunotherapy drug; a use of combination of IFN-γ with an immune checkpoint inhibitor in preparation of a tumor immunotherapy drug; a use of combination of IFN-γ, and T cell preparation with an immune checkpoint inhibitor in preparation of a tumor immunotherapy drug; and a combination of drug combination.

. The other aspect of the present invention also provides a novel CAR expression vector modified with IFN-γ and a construction strategy thereof.

### Beneficial Effects

. The present invention revises the current understanding of IFN-γ in the prior art and finds that IFN-γ enables tumor cells to sensitize the immunotherapy (T cell preparation and/or PD-L1/PD-1 inhibitor) by activating the IFN-γ signaling pathway in tumor cells, for example, by upregulating the expression of ICAM-1 in tumor cells, which inhibits the acquired immunity resistance mediated by PD-L1-PD-1 and enhances the anti-tumor effect of immunotherapy. Thus, the present invention proposes a technical solution that IFN-γ as an adjuvant immunotherapy drug for anti-tumor.

. Interferon (IFN) consists of a cluster of secreted proteins. The IFN protein family is divided into three types, namely type I, type II and type III interferons, based on gene sequence, chromosomal location and receptor specificity thereof. The type II interferon is composed of a single gene family of IFN-γ, also known as immune interferon. Under normal circumstances, the content of IFN-γ in the cells is very low, but the expression level is significantly increased after induction by virus or other interferon inducers. IFN-γ is the most important cytokine involved in anti-tumor immunity, which is an immune-activating cytokine released after CAR-T contacts the antigen. In order to avoid being damaged by activated immune responses, normal tissues can promote the reestablishment of cellular balance in the body under inflammatory conditions by inducing various regulatory pathways. Existing study suggests that tumor cells can use these protective regulatory pathways for immune escape. For example, tumor cells can upregulate the expression of PD-L1 through the release of IFN-γ, thus inhibiting T cells' killing against tumor cells. The acquired immune resistance is considered to be one of the possible reasons that the effective CAR-T-induced killing of the solid tumors is restricted. Considering that upregulation of PD-L1 expression by IFN-γ can induce acquired immune resistance in tumor cells, the existing immunotherapy technical solution does not fully utilize IFN-γ. Even IFN-γ anti-tumor therapeutic solution that entered clinical trials in the early years also failed to become anti-tumor drugs on the market because of poor effect. It is now considered that the IFN-γ upregulates the expression of PD-1, resulting in its poor anti-tumor effect. Therefore, it is considered that it's necessary to use a PD-L1 inhibitor together to neutralize the negative effect that the IFN-γ upregulates the PD-L1 to make IFN-γ produce anti-tumor effect. But this is only a theoretical speculation, and there are no conclusive research results to confirm.

. However, the experimental results of the present invention show that although IFN-γ (the IFN-γ signaling pathway is activated by priming tumor cells) can upregulate PD-L1 expression in tumor cells, IFN-γ also affects the expression of other factors (for example, ICAM-1 expression is upregulated) at the same time. The results show that the IFN-γ significantly enhances the cytotoxicity of CAR-T cells (or other types of genetically engineered T cells) or non-genetically engineered T cells on tumor cells, and IFN-γ can also enhance the sustainable killing ability of CAR-T cells to tumor cells. In other words, the IFN-γ can sensitize tumor cells to T cells, that is, enhancing the sensitivity of tumor cells to T cells. Moreover, the experimental results of the present invention also find that the damage of IFN-γ signaling pathway significantly affects the killing effect of CAR-T to tumor cells.

. Based on the above experimental results and reasonable inference, the present invention proposes a technical solution that IFN-γ as an adjuvant drug combines with T cell preparation to treat solid tumors, and an application of IFN-γ as an adjuvant drug in anti-tumor therapy. Activation of signaling pathway in tumor cell by using the INF-γ can enhance anti-tumor effect of T cell preparation (comprising non-genetically engineered T cells or genetically engineered T cells (e.g., CAR-T and TCR-T). Because the core of the present invention is activation of the IFN-γ signaling pathway in tumor cells, whether for wild-type or mutant IFN-γ and for full-length or fragment of IFN-γ, as long as the IFN-γ signaling pathway in tumor cells can be effectively activated, it can be applied to the present invention, which is within the technical solution of the present invention.

. In addition, because an action principle of PD-L1 inhibitors and/or PD-1 inhibitors is to activate the exhausted T cells to recover their anti-tumor effects, the anti-tumor effects of the PD-L1 inhibitors and/or the PD-1 inhibitors are also essentially mediated by the T cells. Therefore, the present invention also proposes a technical solution to enhance PD-L1 inhibitors and/or PD-1 inhibitors by IFN-γ to inhibit the tumor, and a technical solution to enhance PD-L1 inhibitors and/or PD-1 inhibitors by IFN-γ in combination with T cell preparation to inhibit the tumor. It should be emphasized that the technical solution proposed in the present invention is to use IFN-γ to sensitize tumor cells to assist the enhancement of the anti-tumor effect of immune checkpoint inhibitors (PD-L1 inhibitors and/or PD-1 inhibitors), which is a different technical solution from the use of PD-L1 inhibitors and/or PD-1 inhibitors to inhibit the down-regulation of PD-L1 in tumor cells caused by the activation of the IFN-γ signaling pathway. Also, the former treatment strategy utilizes PD-L1 inhibitors and/or PD-1 inhibitors as the main drug, while the latter treatment strategy utilizes PD-L1 inhibitors and/or PD-1 inhibitors as the adjuvant drug.

. In addition, the experiment of the present invention also shows that the deficiency of the IFN-γ signaling pathway can affect the anti-tumor effect of CAR-T cells. According to research results that deficiency of the IFN-γ signaling pathway can affect the anti-tumor effect of PD-L1 inhibitors and/or PD-1 inhibitors, the present invention also provides a method for determining in advance whether T-cell therapy and/or PD-L1 inhibitors and/or PD-1 inhibitors are an suitable anti-tumor therapeutic option by detecting the activation/deficiency of the IFN-γ signaling pathway (for example, detecting ICAM-1 or IFN-yR2 expression in the tumor cells).

. To sum up, one of the contributions of the technical solution of the present invention is to revise the current understanding of IFN-γ in the prior art and propose an immunotherapy adjuvant drug utilizing IFN-γ as the core component, which enhances the anti-tumor effect of cell therapy and immune checkpoint inhibitors used in combination with cell therapy, and further improves the anti-tumor effect of the existing immunotherapy solution.

. In another aspect, the experimental results of the present invention show that IFN-γ can increase the sensitivity of tumor cells to T cells. For example, IFN-γ can upregulate PD-L1 expression of the tumor cells by priming tumor cells, but the final results show that the IFN-γ significantly enhances the cytotoxicity of CAR-T cells (or other types of genetically engineered T cells, such as TCR-T cells) or non-genetically engineered T cells to the tumor cells. In other words, IFN-γ can sensitize the tumor cells to the T cells, and increases the sensitivity CR of the tumor cells to the T cells.

. On this basis, the present invention proposes a technical solution in which the tumor cells are inhibited by using T cells with high expression of IFN-γ (comprising natural T cells or T cells genetically engineered to have high expression of IFN-γ, as well as genetically engineered T cells (CAR-T cells and TCR-T cells) with high expression of IFN-γ).

. Particularly, the present invention proposes a modification solution for novel CAR-T cells. In addition to the function of activating CAR-T cells, more importantly, this solution can change the immunosuppressive microenvironment in the solid tumors, thereby sensitizing the tumor cells, and producing the stronger anti-tumor effect of CAR-T cell therapy.

. In the technical solution of the present invention, by inserting a segment of an IFN-γ gene into the CAR expression vector, the prepared CAR-T cells can secrete an interferon cytokine IFN-γ. IFN-γ is an important cytokine involved in anti-tumor immunity. It not only can activate CAR-T cells (the CAR-T cells are more activated after being in contact with the tumor cells and more cytokines are released, such as IL-2 and IFN-γ), but also can sensitize the tumor cells when IFN-γ is secreted into the tumor microenvironment, so that the sensitivity of the tumor cells to CAR-T-induced killing is enhanced. More importantly, molecules which are newly highly expressed on IFN-γ-sensitized tumor cells, such as ICAM-1, sensitize tumor cells to immunotherapy (T cell preparation and/or PD-L1/PD-1 inhibitors), thereby inhibiting the inhibition of CAR-T cell activity by PD-L1-PD-1. Therefore, this optimization technology provided by the present invention can further improve the therapeutic effect of CAR-T on solid tumors.

### Description of Drawings

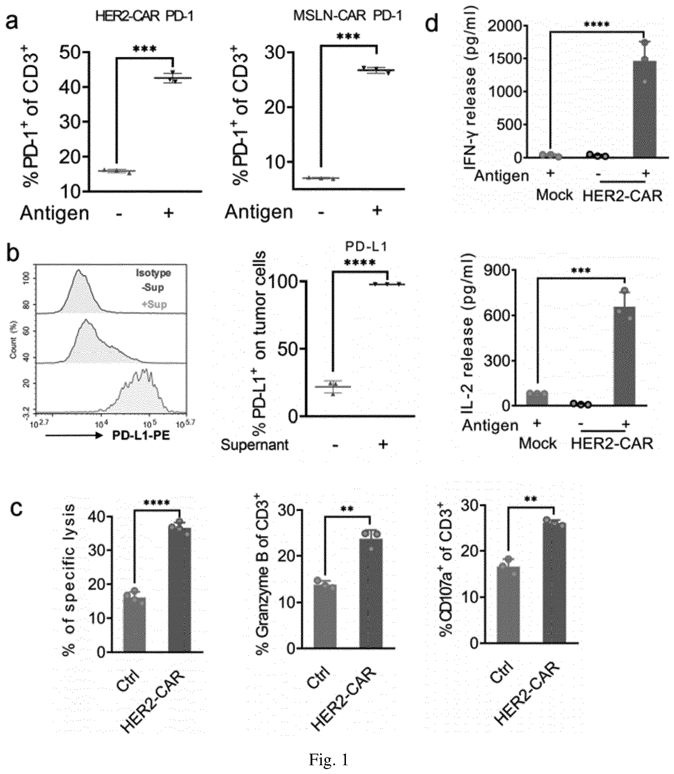

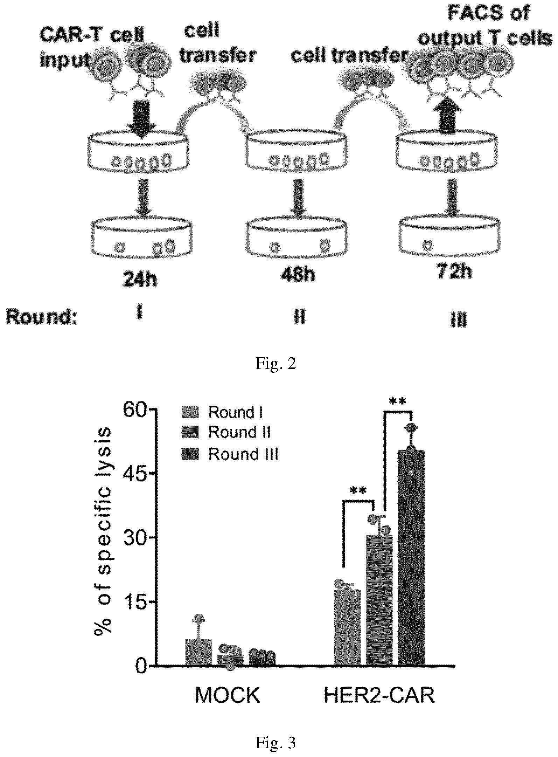

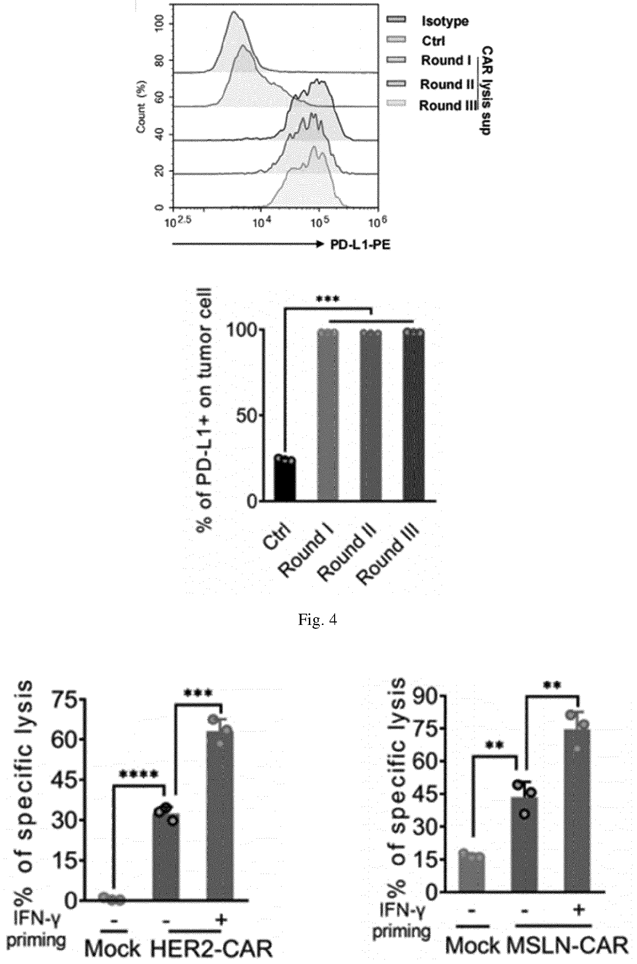

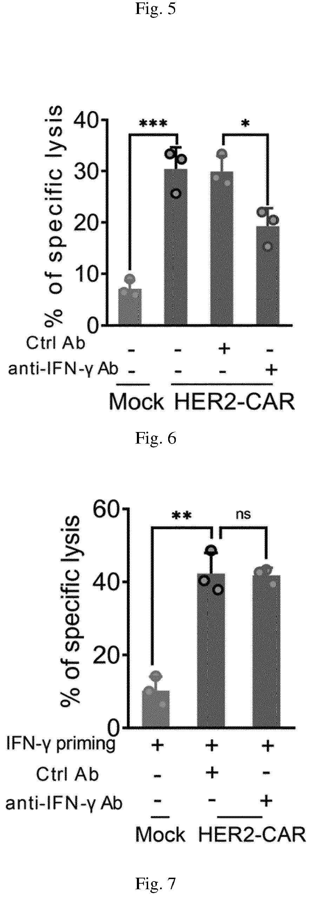

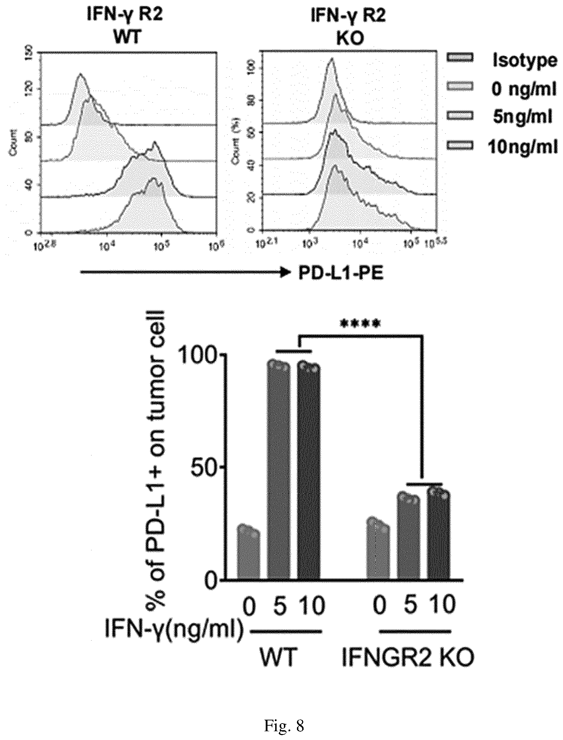

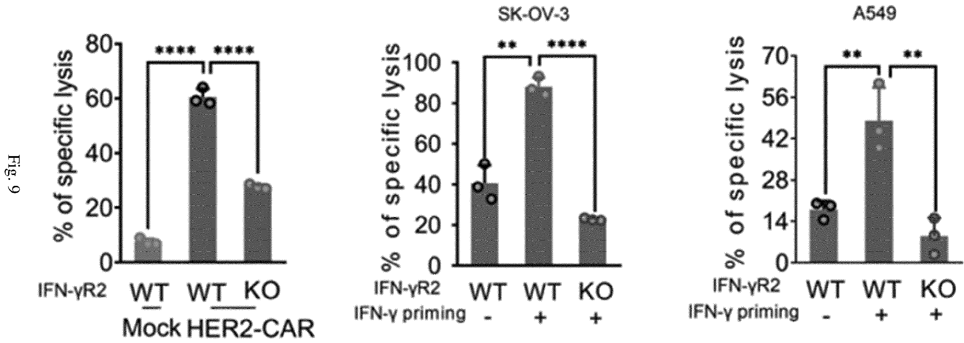

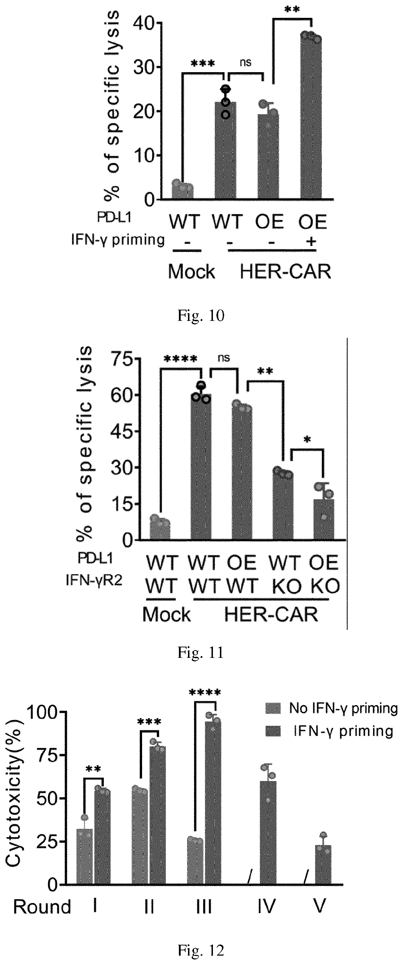

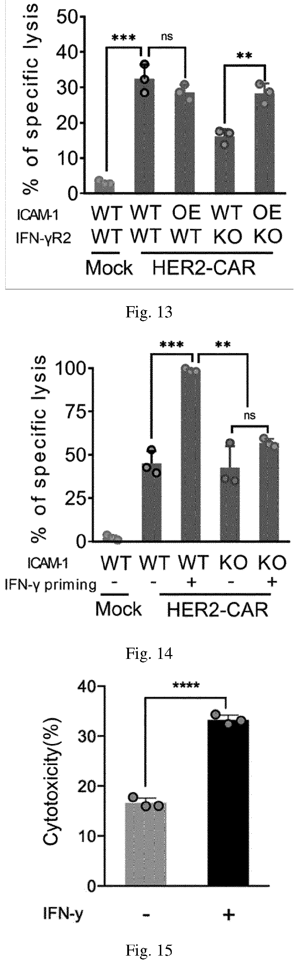

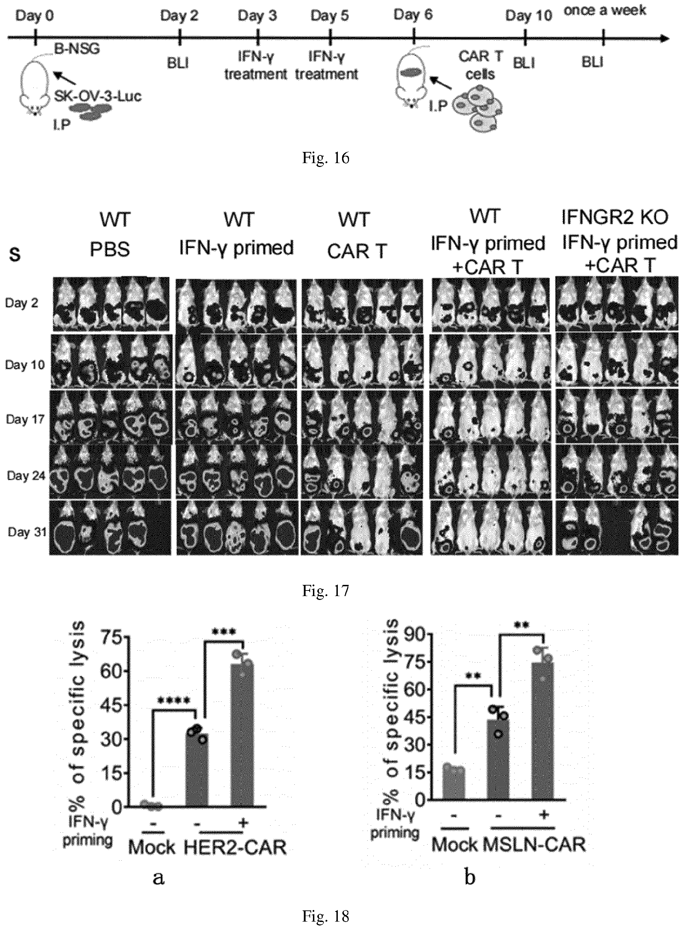

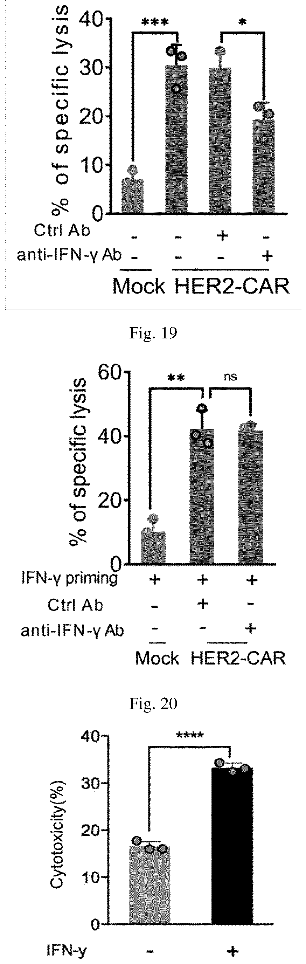

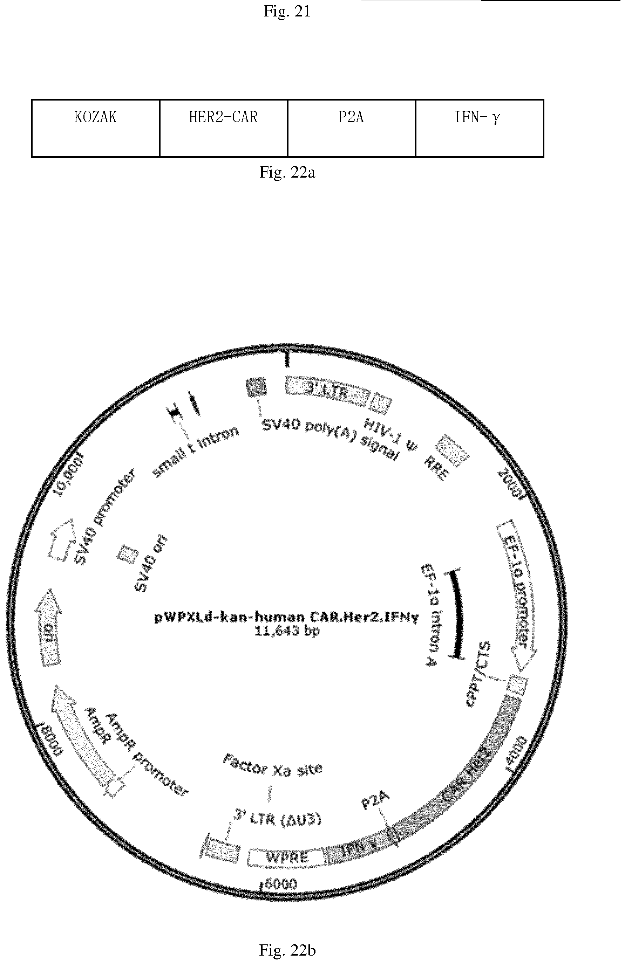

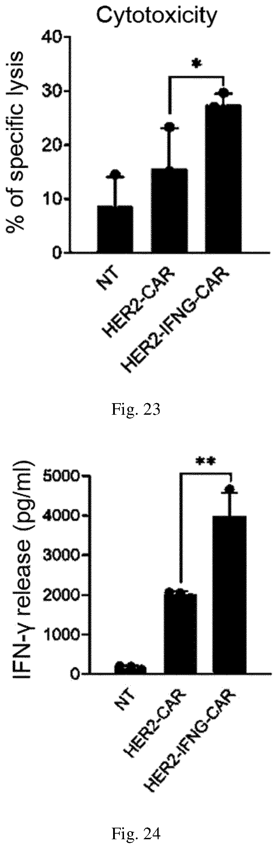

. To make the embodiments of the present invention or the technical solutions in the prior art clearer, the drawings required to be used in the description of the embodiments or the prior art will be briefly introduced below. It is obvious that the drawings described below are some embodiments of the present invention, and that other drawings can be obtained from these drawings for those of ordinary skill in the art without making inventive effort.
. Fig. 1 shows activation of CAR-T cells and expression of PD-L1 and PD-1 when CAR-T kills the solid tumor cells (a shows HER2 (Epidermal Growth Factor Receptor 2)-CAR cells and MSLN (Mesothelin)-CAR cells. Under the condition of antigen stimulation, the expression of PD-1 on CAR-T cells is upregulated. b shows high expression of PD-L1 on the tumor cells. c shows that CAR T cells effectively kill the tumor cells, with high expression of Granzyme B and CD107a. And d shows three kinds of situations: MOCK, HER2-CAR minus and HER2-CAR plus);
. Fig. 2 is a schematic diagram of the experiment mimicking the in vivo antitumor environment where CAR-T cells serially encounter and kill tumor cells;
. Fig. 3 shows that CAR-T cells exhibit higher and higher cytotoxicity and cytokine release ability;
. Fig. 4 shows high expression of PD-L1 on the tumor cells;
. Fig. 5 shows that CAR-T cells exhibit more potent cytolytic activity and IFN-γ secretion ability when the CAR-T cells encounter the IFN-γ-primed tumor cells with high expression of PD-L1;
. Fig. 6 shows that cytotoxicity of CAR-T cells is significantly attenuated when the IFN-γ is neutralized by anti-IFN-antibodies;
. Fig. 7 shows that CAR-T cells still exhibit the ability to kill IFN-γ-primed tumor cells when IFN-γ is neutralized;
. Fig. 8 shows that the knockout IFN-γR2 tumor cells do not express PD-L1 after being primed with IFN-γ (three concentrations: 0ng/ml, 5ng/ml, and10ng/ml);
. Fig. 9 shows that killing effect of CAR-T cells on knockout IFN-γR2 tumor cells is significantly attenuated, and IFN-γ priming cannot enhance the killing effect of CAR-T cells on the knockout IFN-yR2 tumor cells;
. Fig. 10 shows that overexpression of PD-L1 on the tumor cells does not make the tumor cells inhibit the CAR-T-induced killing, and not inhibit the enhancement of the cytotoxicity of the CAR-T cells by IFN-γ priming;
. Fig. 11 shows that high expression of PD-L1 on the knockout-IFN-γR2 tumor cells can significantly inhibit the CAR-T-induced killing to the tumor cells;
. Fig. 12 shows effect of the CAR-T cells on IFN-γ primed tumor cells in serial in vitro killing experiments;
. Fig. 13 shows that high expression of ICAM-1 in the knockout INF-γR2 tumor cells enhances the killing effect of CAR-T cells;
. Fig. 14 shows that IFN-γ priming cannot enhance the killing effect of CAR-T cells on the tumor cells that INF-γR2 gene is knocked out;
. Fig. 15 is a diagram showing the effect that the IFN-γ enhances the killing ability of ordinary T cells;
. Fig. 16 is a schematic diagram of a combinational therapy of IFN-γ and CAR-T in peritoneal ovarian cancer model in NSG mice;
. Fig. 17 shows the effect of a combinational therapy of IFN-γ and CAR-T in peritoneal ovarian cancer model in NSG mice;
. Fig. 18 shows that CAR-T cells exhibit more potent cytolytic activity and IFN-γ secretion ability when CAR-T cells encounter IFN-γ-primed tumor cells with high expression of PD-L1; (in Fig. 18a, the CAR-T cell is HER2-CAR; and in Fig. 18b, the CAR-T cell is MSLN-CAR);
. Fig. 19 shows that the cytotoxicity of CAR-T cells is significantly attenuated when the IFN-γ is neutralized by anti-IFN-antibodies;
. Fig. 20 shows that CAR-T cells still exhibit the ability to kill IFN-γ-primed tumor cells under the condition that IFN-γ secreted by CAR-T cells is neutralized;
. Fig. 21 is a diagram of the effect that IFN-γ-sensitized tumor cells enhance the killing ability of ordinary T cells;
. Fig. 22 is a diagram of a plasmid vector skeleton (22a is a reference diagram of structure and positional relationship of each element on an expression cassette; and 22b is a reference diagram of a plasmid vector skeleton);
. Fig. 23 shows detection of the effect that the CAR-T cells kill the tumor cells; and
. Fig. 24 shows detection of secretion of cytokine IFN-γ when the CAR-T cells kill the tumor cells.

### Detailed Description

. To make the objective, the technical solutions and advantages of the embodiments of the present invention clearer, the technical solutions of the embodiments of the present invention will be clearly and completely described below in combination with drawings. It is obvious that the described embodiments are some of the embodiments of the present invention, not all of the embodiments. Based on the embodiments of the present invention, all other embodiments obtained by those of ordinary skill in the art without making inventive effort shall belong to the protection scope of the present invention.

. The embodiments are for the purpose of better illustration of the present invention, but the contents of the present invention are not limited to the embodiments. Therefore, non-essential improvements and adjustments of the embodiments made by those skilled in the art according to the above-mentioned contents of the present invention still belong to the protection scope of the present invention.

. It should be noted that the term "include", "comprise" or any variant thereof is intended to encompass nonexclusive inclusion so that a process, method, article or device including a series of elements includes not only those elements but also other elements which have been not listed definitely or an element(s) inherent to the process, method, article or device. Moreover, the expression "comprising a(n) ... " in which an element is defined will not preclude presence of an additional identical element(s) in a process, method, article or device comprising the defined element(s) unless further defined.

. As used herein, the term "about", typically means +/-5% of the stated value, more typically +/-4% of the stated value, more typically +/-3% of the stated value, more typically, +/-2% of the stated value, even more typically +/-1% of the stated value, and even more typically +/-0.5% of the stated value.

. Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

### Noun definitions:

. An "adjuvant drug" mentioned in the present invention refers to a drug used for assisting to enhance the efficacy of a main therapeutic drug (i.e., main drug (primary drug)). For example, the IFN-γ mentioned in the present invention can be applied to the patient's body as priming drug prior to the treatment of a main drug, to increase the sensitivity of tumor cells to the subsequent main drug, thereby enhancing the anti-tumor effect of the subsequent main drug. For example, IFN-γ mentioned in the present invention can also be used as priming drug to upregulate ICAM-1 expression in tumor cells and enhance the killing ability and cytokine release of CAR-T cells or non-genetically engineered T cells on tumor cells. Of course, an adjuvant drug is not necessarily used before the treatment of a main drug, but can also be used together with a main drug according to the actual situation.

. The "sensitization" mentioned in the present invention refers to the increase of the sensitivity of tumor cells to a main drug (immunotherapy) by an adjuvant drug, which facilitates the efficacy of a main drug and more effective in killing tumor cells. For example, the present invention enhances the sensitivity of tumor cells to T cells (comprising T cells in vivo and adoptively infused T cell preparations) and the sensitivity to immune checkpoint inhibitors (PD-L1 inhibitors and/or PD-1 inhibitors) by activating the IFN-γ signaling pathway in tumor cells through the IFN-γ. Therefore, the immunotherapy based on T cells and/or immune checkpoint inhibitors can achieve better results.

. The "sensitization" mentioned in the present invention also refers to the increase of the sensitivity of tumor cells to immunotherapy by changing immunosuppression microenvironment in the tumor through a tumor cell sensitizing factor (such as wild type or mutant IFN-γ), thereby more effectively killing the tumor cells.

### A invention: use of IFN-γ in preparation of an anti-tumor drug

. A invention belongs to the field of biomedicine, and particularly relates to an anti-tumor drug for assisting anti-tumor immunotherapy and an application thereof.

. Tumor immunotherapy is a revolutionary progress in anti-tumor therapy, which can fight tumors by mobilizing a body's own immune function. The CAR-T therapy belongs to the field of immunotherapy. The CAR-T cell therapy has achieved surprising anti-tumor effect in the treatment of hematologic tumors by targeting CD19. By April 2018, there are a total of 2,513 immunotherapies for the tumors, 382 in China, and 227 in clinical phases III and IV. Kymriah (Novartis company) and Yescarta (Kite company) that have been on the market are respectively used for treating childhood acute lymphoblastic leukemia and adult B-cell lymphoma. The complete response (CR) of Kymriah is 82.5%, and the relapse probability is 75% by 6 months and is 64% by 12 months. For another FDA-approved CD19 specific CAR-T product, Yescarta, can achieve 43 to 52% of CR in treating patients with relapsed or refractory DLBCL.

. However, for solid tumors, the therapeutic effect of CAR-T is not very good. For example, in phase I clinical studies of EGFR-CAR-T in the treatment of epidermal growth factor receptor cells in relapsed/refractory EGFR + non-small cell lung cancer, there were only 2 respondents and 5 patients with stable disease (SD) among 11 cancer patients. The feasibility and safety of administering HER2-CAR-T cells to patients with relapsed or refractory HER2-positive tumors were evaluated in phase I/II studies of a dose-escalation clinical trial. However, the clinical benefits were limited, with only 4/17 evaluable patients showing SD. That is, the therapeutic effect of CAR-T cells in solid tumors is limited. How to enhance and promote the therapeutic effect of CAR-T cells on solid tumors is an urgent problem to be solved in the prior art.

. As an inhibitory molecule in costimulatory signals, the main function of PD-1 was first discovered to induce apoptosis of T cell. PD-1 can negatively regulate T cell-mediated immune responses by binding to its ligand, programmed death ligand 1 (PD-L1), to help tumor cells "escape". Therefore, blocking this signaling pathway is considered as an effective cancer immunotherapy. Since the approval of the first PD-1 inhibitors, pembrolizumab and nivolumab, in 2014, there has been unprecedented growth in the clinical research of PD-1/PD-L1 inhibitors as a form of cancer immunotherapy. PD-1 and PD-L1 inhibitors in clinical trials can be used for various types of cancers, comprising advanced melanoma, non-small cell lung cancer, renal cell carcinoma, bladder cancer, hodgkin lymphoma, liver cancer, head and neck squamous cell carcinoma, urothelial carcinoma, Merkel cell carcinoma, etc. So far, there are three PD-1 inhibitors that have been on the market abroad: Pembrolizumab, Nivolumab and Cemiplimab; and there are four inhibitors that have been on the market in China: Toripalimab, Tislelizumab, Tyvyt and Camrelizumab. The PD-L1 inhibitors that have been on the market are respectively: Atezolizumab, Avelumab and Durvalumab. When PD-L1/PD-1 therapy is applied to more solid tumors, how to make the PD-L1/PD-1 therapy achieve better anti-tumor therapeutic effect is a problem to be solved at present.

. To sum up, how to improve the anti-tumor effect of existing immunotherapy such as immunotherapy and/or immune checkpoint inhibitor therapy and propose a more optimized and more effective anti-tumor therapeutic solution are very important problems in the field of tumor immunotherapy at present, and are also technical problems that A invention tries to address.

. A purpose of the A invention is to provide a use of IFN-γ in preparation of an anti-tumor adjuvant drug; a kit used for detecting anti-tumor effect of an immune checkpoint inhibitor; an anti-tumor drug for assisting anti-tumor immunotherapy; a use of combination of IFN-γ with T cell preparation in preparation of an tumor immunotherapy drug; a use of combination of IFN-γ with an immune checkpoint inhibitor in preparation of a tumor immunotherapy drug; a use of combination of IFN-γ, and T cell preparation with an immune checkpoint inhibitor in preparation of a tumor immunotherapy drug; and a combination of drug combination.

. To achieve above purposes, the technical solution of A invention is:
. Use of IFN-γ in preparation of an anti-tumor adjuvant drug, the anti-tumor adjuvant drug is used for assisting treatment of a T cell preparation, wherein the IFN-γ enhances killing effect of the T cell preparation on tumor cell by sensitizing the tumor cell; the T cell preparation comprises non-genetically engineered T cell and/or genetically engineered T cell; and the IFN-γ comprises full-length or fragment of wild-type or mutant IFN-γ.

. Further, the genetically engineered T cell comprises CAR-T cell and/or TCR-T cell.

. Further, the IFN-γ sensitizes the tumor cell by activating an IFN-γ signaling pathway of the tumor cell.

. Further, activation of IFN-γ signaling of the tumor cell upregulates ICAM-1 and enhances the killing effect of the T cell preparation on the tumor cell.

. Further, the tumor for which the anti-tumor adjuvant drug is used is solid tumor.

. Further, the tumor for which the anti-tumor adjuvant drug is used is ovarian tumor, breast cancer, brain gliomas, gastric cancer, colon cancer, melanoma, non-small cell lung cancer, renal cell carcinoma, bladder cancer, liver cancer or urothelial carcinoma.

. A invention also provides use of IFN-γ in preparation of an anti-tumor adjuvant drug, the anti-tumor adjuvant drug is used for assisting treatment of an immune checkpoint inhibitor, wherein the IFN-γ assists anti-tumor effect of the immune checkpoint inhibitor by sensitizing the tumor cell, and the immune checkpoint inhibitor comprises a PD-L1 inhibitor and/or a PD-1 inhibitor; and the IFN-γ comprises full-length or fragment of wild-type or mutant IFN-γ.

. Further, the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

. Further, the tumor for which the anti-tumor adjuvant drug is used is solid tumor.

. Further, tumor for which the anti-tumor adjuvant drug is used is ovarian tumor, breast cancer, brain gliomas, gastric cancer, colon cancer, melanoma, non-small cell lung cancer, renal cell carcinoma, bladder cancer, liver cancer or urothelial carcinoma.

. A invention also provides use of IFN-γ in preparation of an anti-tumor adjuvant drug, the anti-tumor adjuvant drug is used for assisting treatment of a T cell preparation and an immune checkpoint inhibitor, wherein the IFN-γ assists anti-tumor effect of the T cell preparation and the immune checkpoint inhibitor by sensitizing the tumor cell; the immune checkpoint inhibitor comprises a PD-L1 inhibitor and/or a PD-1 inhibitor; the T cell preparation comprises non-genetically engineered T cell and/or genetically engineered T cell; and the IFN-γ comprises full-length or fragment of wild-type or mutant IFN-γ.

. Further, the genetically engineered T cell comprises CAR-T cell and/or TCR-T cell.

. Further, the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

. Further, the anti-tumor adjuvant drug stimulates the tumor cell to produce ICAM-1 and enhance killing ability of the T cell preparation on the tumor cell.

. Further, the tumor is solid tumor.

. Further, the tumor is ovarian tumor, breast cancer, brain gliomas, gastric cancer, colon cancer, melanoma, non-small cell lung cancer, renal cell carcinoma, bladder cancer, liver cancer or urothelial carcinoma.

. A invention also provides a kit used for detecting anti-tumor effect of an immune checkpoint inhibitor, the immune checkpoint inhibitor comprises a PD-L1 inhibitor and/or a PD-1 inhibitor, wherein the kit comprises detecting whether there is any obstruction of IFN-γ signaling pathway of tumor cell.

. Further, the kit comprises detecting an expression level of ICAM-1 or IFN-γR2 in the tumor cell. It should be noted that the purpose of detecting an expression level of ICAM-1 or IFN-γR2 is to detect whether there is any obstruction of IFN-γ signaling pathway. Thus, no matter whether full-length or fragment of ICAM-1 and IFN-γR2 (wild-type or mutant) is used for detection, as long as they can be utilized to effectively detect the obstruction of IFN-γ signaling pathway, they are within the protection scope of the present invention.

. Further, the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

. Further, the tumor is solid tumor.

. Further, the tumor is ovarian tumor, breast cancer, brain gliomas, gastric cancer, colon cancer, melanoma, non-small cell lung cancer, renal cell carcinoma, bladder cancer, liver cancer or urothelial carcinoma.

. A invention also provides an anti-tumor drug for assisting anti-tumor immunotherapy, wherein the anti-tumor drug comprises full-length or fragment of wild-type or mutant IFN-γ, and assists anti-tumor effect of a T cell preparation and/or an immune checkpoint inhibitor by sensitizing tumor cell.

. Further, the anti-tumor drug comprises targeting vector, and the targeting vector can transport the full-length or fragment of wild-type or mutant IFN-γ to the tumor cell.

. Further, the anti-tumor drug is used together with the T cell preparation and/or the immune checkpoint inhibitor.

. Further, the anti-tumor drug further comprises any pharmaceutically acceptable vector and/or auxiliary.

. A invention also provides an anti-tumor drug, and the anti-tumor drug comprises an anti-tumor composition comprising wild-type or mutant IFN-γ and a T cell preparation.

. A invention also provides an anti-tumor composition, and the anti-tumor composition comprises wild-type or mutant IFN-γ and a PD-L1 inhibitor and/or a PD-1 inhibitor and a T cell preparation.

### Detailed Description of A Invention:

. The experiment of the present invention shows that the up-regulation of both PD-L1 and PD-1 co-exists with the potent activation, cytolysis capability (killing of tumor cells) and cytokines secretion of CAR-T cells during CAR-T-induced killing of solid tumor cells (Fig. 1). Two kinds of CAR-T cells are used in Fig. 1a: Epidermal Growth Factor Receptor 2 (HER2)-CAR cells and MSLN (Mesothelin)-CAR cells, and the expressions of PD-1 are upregulated in both CAR-T cells under the condition of antigen stimulation. The antigens are tumor cells that express HER2 or MSLN. In Fig. 1b, the CAR-T cells are co-cultured with the tumor cells and accordingly are activated to kill the tumor cells. The coculture of the CAR-T cells and the tumor cells is centrifuged to remove the cell debris and collect the "supernatant". The supernatant is deemed as the "microenvironment" wherein the activated CAR-T cells act on other tumor cells. In Fig. 1c, the lysis percentage of the tumor cells is higher after the CAR-T cells are added. And both Granzyme B and OD107a+ on the CAR-T cells are upregulated, indicating that CAR-T cells are activated to play a role in killing tumor cells. There are three groups of experiments in Fig. Id: MOCK, HER2-CAR minus and HER2-CAR plus, wherein the MOCK is control T cells that do not express CAR structures. In Fig. 1, the CAR-T cells and tumor cells are co-incubated, and all tumor cells are killed by CAR-T after 24 hours. The supernatant is obtained by centrifugation, and new tumor cells are cultured in the supernatant, and the expression of PD-L1 on the tumor cells is detected.

. T-cell exhaustion markers TIM-3 and LAG-3 are significantly increased on the CAR-T cells. In the stress test of mimicking the in vivo antitumor environment where CAR-T cells serially encounter and kill tumor cells (Fig. 2), CAR-T cells show increasingly higher cytotoxicity and cytokines releasing, despite high expression of PD-L1 on the tumor cells (Fig. 3). These results indicate that CAR-T per se has an inhibitory effect on PD-L1-PD-1 during CAR-T-induced killing of tumor cells.

. Subtoxic concentration of IFN-γ induces high expression of PD-L1 on the tumor cells. However, when CAR-T cells encounter the IFN-γ-primed tumor cells with high expression of PD-L1, CAR-T cells exhibit more potent cytolytic activity and IFN-γ secretion (Fig. 5). On the contrary, when IFN-γ is neutralized by anti-IFN-γ antibody, the cytotoxicity of CAR-T cells is significantly attenuated (Fig. 6), indicating that the IFN-γ is critical to CAR-T activities.

. In IFN-γ primed tests, the media is replaced with fresh media making sure that residual IFN-γ is undetectable (less than 10pg/ml), excluding the direct effect of IFN-γ to CAR-T cells.

. The present invention primed tumor cells with low-concentration IFN-γ, and then detects the cytotoxicity of CAR-T cells in the fresh media containing sufficient anti-IFN-γ antibody to neutralize IFN-γ from CAR-T. When the IFN-γ is neutralized, CAR-T cells still exhibit the similar killing ability towards tumor cells primed with low-concentration IFN-γ (Fig. 7). This is in contrast to tumor cells without IFN-γ-priming, which can resist CAR-T-induced killing effects (Fig. 6). Therefore, it is considered that IFN-γ enhances the killing ability of CAR-T by acting on tumor cells. In other words, priming with IFN-γ enhances the sensitivity of tumor cells to CAR-T cells.

. Then, the IFN-γ signaling pathway is blocked by using a CRISPR-Cas9 system, to disrupt the expression of an IFN-yR2 (IFNGR2) gene in the tumor cells. Because IFN-γR1 is ubiquitous in mammalian cells, but IFN-γR2 is more dynamically expressed and is considered to determine the extent of IFN-γ-induced signaling in specific cell populations. The knockout-IFN-γR2 (IFN-γR2-null) tumor cells grow as normally as control cells and do not express the PD-L1 any more under the treatment of IFN-γ (three concentrations: 0ng/ml, 5ng/ml and 10ng/ml) (Fig. 8). However, the knockout-IFN-γR2 tumor cells are more resistant to the cytolysis of CAR-T cells (Fig. 9). In two different tumor cells (SK-OV-3, A549), the enhancement of CAR-T cell cytotoxicity by IFN-γ priming is significantly inhibited (Fig. 9). That is, the effect of sensitizing tumor cells by IFN-γ priming is inhibited. These results jointly indicate that the effect of IFN-γ to tumor cells represents an intrinsic mechanism of CAR-T to sustain or enhance cytolytic activity (and anti-tumor effect) under the conditions of PD-L1 and PD-1 expressions.

. In order to directly characterize the inhibitory effect of the IFN-γ on the PD-L1-PD-1, in this experimental group, CAR-T activities to tumor cells with stable expression of PD-L1are detected. The overexpression of PD-L1 on the tumor cells do not make the tumor cells resistant to CAR-T-induced killing, and also do not inhibit the enhancement of CAR-T's cytotoxicity to IFN-γ-primed tumor cells (Fig. 10). In other words, IFN-γ priming enhances the sensitivity of the tumor cells to the CAR-T cells, and this will not be inhibited by PD-L1. However, in the knockout IFN-γR2 tumor cells, the high expression of PD-L1 can significantly inhibit the killing effect of the CAR-T cells (Fig. 11). These results indicate that the IFN-γ signaling can surmount the inhibitory effect of the PD-L1-PD-1, enhance the sensitivity of the tumor cells to the CAR-T cells, and enhance CAR-T activities.

. Next, the present invention also investigates how the IFN-γ surmounts the inhibitory effect of the PD-L1-PD-1 to CAR-T. In addition to inducing the PD-L1 expression, the IFN-γ will also induce the tumor cells to express HLA molecules. HLA is a natural ligand of TCRs for T cells activation. However, the blocking to the HLA-ABC molecules do not affect the enhancement effect of the IFN-γ to the CAR-T cells.

. An intercellular adhesion molecule 1 (ICAM-1, also known as CD54) is a cell surface glycoprotein on antigen presenting cells (APCs) and plays an important role during an effective immune response. The researches in the present invention find that the knockout of ICAM-1 in the tumor cells that have functional IFN-γ receptors almost completely eliminates the enhancement of CAR-T's cytotoxicity to IFN-γ-primed tumor cells (Fig. 14), indicating the importance of ICAM-1 for CAR-T activity. At the same time, IFN-γ stimulates tumor cells to produce the ICAM-1, which can enhance the killing ability of the T cell against the tumor cells (Fig. 15). On the contrary, overexpression of ICAM-1 in the tumor cells with a normal IFN-γ signaling pathway do not significantly promote the specific cytolysis of CAR-T to tumor cells (Fig. 13). It is speculated that this is because of the elevation of ICAM-1 induced by the IFN-γ released from activated CAR-T cells. Therefore, the present invention investigates the overexpression of ICAM-1 in IFN-γR2 knockout cells, and finds that ICAM-1 overexpression make IFN-γR2-deficient tumor cells are more vulnerable to CAR-T-induced killing (Fig. 13). These results jointly indicate that IFN-γ-induced ICAM-1 expression inhibits PD-L1-PD-1 functions and enhances anti-tumor activities of CAR-T cells and T cells.

. Then, the experiment in the present invention test whether the IFN-γ priming can help a CAR-T cell preparation achieve better therapeutic effect to solid tumors. Serial killing experiments in vitro show that CAR-T cells keep cytolytic activity to IFN-γ-primed tumor cells until round 5, whereas only until round 3 to non-IFN-γ primed tumor cells (Fig. 12). In other words, IFN-γ (as an adjuvant drug) priming can enhance the sensitivity of tumor cells to the CAR-T cells, which is beneficial to the therapeutic effect of the CAR-T cell preparation as the main drug on the solid tumors.

. Then, in the experiment of the present invention, a peritoneal ovarian cancer model in NSG mice is established. The IFN-γ is administered twice before CAR-T cells are intraperitoneally injected (Fig. 16). Bioluminescence imaging show that the IFN-γ has no significantly inhibitory effect on tumor growth, and CAR-T applied alone can only delay tumor growth, whereas the combination of IFN-γ priming and CAR-T cells can lead to sustainable elimination of tumors in three of five mice. And the tumors of the remaining two mice are also significantly smaller than those in the mice of control group (Fig. 17). However, when IFN-γR2 is deficient, tumors are more resistant to the combinational therapy (Fig. 17). These in vitro and in vivo experiment results indicate that the sequential therapy using IFN-γ (as an adjuvant drug for sensitizing) and CAR-T (as a main drug) will be an effective method for treating solid tumors.

. To sum up, the experiment in the present invention shows that:
1) after the CAR-T cells contact the tumor cells, the inhibitory molecule PD-1 is highly expressed on CAR-T cells and PD-L1 is highly expressed on the tumor cells. At the same time, the activation markers granzyme B and perforin and cytokine release in the CAR-T cells are up-regulated, and the CAR-T effectively kills the tumor cells.
2) By priming tumor cells with IFN-γ, IFN-γ upregulates PD-L1 expression on the tumor cells and enhance (not inhibit) the CAR-T-induced killing of tumor cells and cytokine release of CAR-T at the same time. (IFN-γ is low in concentration, and has no significant inhibition on the tumor cells).
3) After IFN-γ in the system which the CAR-T cells are co-incubated with the tumor cells is neutralized using IFN-γ neutralizing antibody, the killing function of CAR-T cells significantly decreases. After knockout of an IFN-γ receptor gene (IFNγR2) in the tumor cells, the tumor cells do not express the PD-L1, but the killing function of the CAR-T decreases.
4) Overexpression of PD-L1 on the tumor cells does not significantly inhibit CAR-T activities, but the overexpression of the PD-L1 on the IFN-γ receptor gene (IFNγR2) knockout tumor cells significantly inhibits the killing function of the CAR-T.
5) The IFN-γ induces high expression of ICAM-1 in the tumor cells. After the ICAM-1 in the tumor cells is knocked out, the IFN-γ priming cannot enhance the killing activity of the CAR-T.
6) ICAM-1 molecules are overexpressed in the IFN-yR2-deficient tumor cells, and the killing function and cytokine release ability of CAR-T are enhanced.
7) In a mouse model of peritoneal ovarian cancer, IFN-γ can enhance the therapeutic effect of the CAR-T cells.
8) Priming tumor cells with IFN-γ promotes ICAM-1 expression in the tumor cells, so that the killing induced by ordinary T cells against the tumor cells can be enhanced.

### . B invention: a CAR expression vector and an application thereof

. The B invention belongs to the field of bioengineering, and particularly relates to a CAR expression vector and an application thereof.

. An adoptive cell therapy is an effective anti-cancer strategy, known as "living drug", which plays an important role in cancer immunotherapy. A chimeric antigen receptor T cell (CAR-T) is a genetically engineered T cell, which has achieved remarkable effect in the treatment of hematological malignancies. However, the therapeutic effect of CAR-T on solid tumors is not satisfactory, and it still faces great challenges. Although the reasons for the poor efficacy of CAR-T cells on solid tumors are not clear at present, there are several hypothesized reasons, such as poor survival of CAR-T cells in vivo which affects their killing efficacy due to their short-lifespan after input into the body; solid tumors can form an immunosuppressive microenvironment due to pluripotency in metabolism, immune escape, and tissue formation, which makes it difficult for CAR-T cells to infiltrate into solid tumor tissue to exert their efficacy. Even when the CAR-T cells enter the solid tumors, the CAR-T cells will be inactivated and die due to the restriction of immune system in many aspects. Therefore, how to improve the killing efficacy of the CAR-T cells and related pharmaceutical preparations on solid tumors is an urgent problem to be solved in this field.

. In order to enhance the therapeutic effect of CAR-T cells, the current strategies mainly focus on further genetic engineering or nanomodification for CAR-T cells. For example, the insertion of multi-target CAR genes into CAR-T cells, or enabling CAR-T cells to express chemokines or direct modification of adhesion molecules or chemical groups to enhance the orientation and recognition of the CAR-T cells to tumor tissue. However, there are some problems and inadequacies in these technical means more or less. Taking multi-target CAR-T cells as an example, excessive insertion of foreign genes can affect the original activity of T cells. And chemical modifications usually result in decreased cell viability and impaired mobility and migration. Therefore, the modification to the CAR-T cells must comprehensively consider multiple positive and negative factors. More targeted and more effective modification methods are selected.

. In the existing technical route, it is considered as a feasible way to modify CAR-T from the perspective of PD-1 inhibition. PD-1 is an important inhibitory molecule in costimulatory signals. PD-1 negatively regulates T cell-mediated immune responses by binding its ligand programmed death ligand 1 (PD-L1). Therefore, blocking the PD-L1-PD-1 signal pathway is an effective anti-tumor immunotherapy strategy, which has clinically achieved good therapeutic effect. At present, a number of antibody drugs targeting PD-L1/PD-1 have been on the market, for example, for the antibodies of the PD-1: Pembrolizumab, Nivolumab and Cemiplimab; and for the antibodies of the PD-L1: Atezolizumab, Avelumab and Durvalumab.

. Inhibitory signaling pathways such as PD-1are also important factors affecting the therapeutic effect of CAR-T. Researches show that blocking the PD-1 pathway can improve the anti-tumor effect of CAR-T cells. Therefore, in the existing technical route, an attempt to make CAR-T cells secrete PD-1 or PD-L1 antibodies by genetic engineering can significantly improve the therapeutic effect on solid tumors, or knocking out the gene encoding the PD-1 in CAR-T cells by gene editing can also improve the anti-tumor activity of CAR-T.

. However, all of these technical solutions and strategies of technical routes focus on improving the functions of the CAR-T cells per se, which have little effect on tumor cells and an immunosuppressive microenvironment in the solid tumors. In other words, current technical solutions and strategies of technical routes are limited because of focusing only on improving the CAR-T cells per se rather than bidirectionally improving the anti-tumor effect of CAR-T cell therapy from the perspective of the tumor cells.

. Therefore, B invention attempts to provide a novel CAR-T cell preparation that can affect tumor cells and an immunosuppressive microenvironment in the solid tumors, to optimize the anti-tumor effect of the CAR-T cell preparation.

. One of the purposes of B invention is to provide a novel CAR expression vector and a construction strategy.

. To achieve above purposes, the technical solution of B invention is:
. A CAR expression vector, comprising nucleic acid encoding chimeric antigen receptor (CAR) and nucleic acid encoding tumor cell sensitizing factor, wherein the nucleic acid encoding tumor cell sensitizing factor is nucleic acid encoding full-length or fragment of wild-type or mutant IFN-γ.

. Further, the nucleic acid encoding CAR is linked to the nucleic acid encoding tumor cell sensitizing factor by a sequence encoding self-cleaving peptide.

. Further, the nucleic acid encoding CAR comprises nucleic acid encoding single-chain antibody variable region (ScFv) targeted tumor-specific antigen, CD8a signal peptide, CD8a hinge region, CD8a transmembrane domain, 4-1BB intracellular costimulatory element and CD3ζintracellular domain.

. Further, the tumor-specific antigen comprises one or more of HER2, Mesothelin, GPC-3, EGFRvIII, MUC1, CD19, CD30, BCMA, EGFR, CD133, PSCA, GD2 and LewisY.

. Further, the sequence encoding self-cleaving peptide is a P2A sequence.

. Further, sequence of the nucleic acid encoding chimeric antigen receptor CAR is shown as SEQ ID NO:1; sequence of the nucleic acid encoding IFN-γ is shown as SEQ ID NO:2; and the sequence encoding self-cleaving peptide is shown as SEQ ID NO:3.

. Another purpose of the present invention is to provide a lentivirus.

. A lentivirus comprising the above CAR expression vector.

. In one embodiment of the present invention, the lentivirus is preferably pWPXLd. However, the lentiviral expression plasmids commonly used in the art, including psPAX2, pMD2.G, and pVSVG, can also be included in the technical solution of the present invention.

. The purpose of the present invention is also to provide a novel CAR-T cell and preparation thereof, and a novel T cell and preparation thereof.

. A CAR-T cell, wherein the CAR-T cell is transfected with vector as described in (a) or (b): (a) the above CAR expression vector; and (b) a CAR expression vector comprising nucleic acid encoding CAR and nucleic acid encoding IFN-γ.

. An anti-tumor drug, wherein the anti-tumor drug comprises the above CAR-T cell and pharmaceutically acceptable adjuvant and/or auxiliary.

. Further, the tumor is solid tumors.

. Further, the tumor is ovarian tumor, breast cancer, brain gliomas, gastric cancer, colon cancer, melanoma, non-small cell lung cancer, renal cell carcinoma, bladder cancer, liver cancer or urothelial carcinoma.

. A T cell, wherein the T cell expresses CAR and IFN-γ.

. Further, the T cell comprises nucleic acid encoding the CAR and nucleic acid encoding the IFN-γ.

. An anti-tumor drug, wherein the anti-tumor drug comprises a T cell expressing CAR and IFN-γ and pharmaceutically acceptable adjuvant and/or auxiliary.

. Further, the tumor is solid tumor.

. Further, the tumor is ovarian tumor, breast cancer, brain gliomas, gastric cancer, colon cancer, melanoma, non-small cell lung cancer, renal cell carcinoma, bladder cancer, liver cancer or urothelial carcinoma.

. A preparation method for T cell expressing CAR and IFN-γ, wherein the preparation method uses vector to transfect nucleic acid encoding CAR and nucleic acid encoding IFN-γ into the T cell.

### . Detailed Description of B Invention

### . Embodiment 1 of B invention: IFN-γ increases the sensitivity of the tumor cells to the immunotherapy

. The tumor cells are induced to overexpress PD-L1 using a subcytotoxic concentration of IFN-γ (that is, the tumor cells are primed with IFN-y). When CAR-T cells (HER2-CAR, MSLN-CAR) interact with the IFN-γ-primed tumor cells with high expression of PD-L1, CAR-T cells exhibit more potent cytolytic activity and IFN-γ secretion ability (Fig. 18). In IFN-γ primed tests, the media is replaced with fresh media making sure that residual IFN-γ is undetectable (less than 10pg/ml), excluding the direct effect of IFN-γ to CAR-T cells.

. In addition, the present invention also adopts a low concentration of IFN-γ to prime tumor cells, and then detects the cytotoxicity of CAR-T (HER2-CAR) cells in the fresh media containing sufficient anti-IFN-γ antibody to neutralize IFN-γ from CAR-T. When IFN-γ is neutralized, CAR-T cells still exhibit the similar killing ability towards tumor cells primed with low-concentration IFN-γ (Fig. 20). This is in contrast to tumor cells without IFN-γ-priming, which can resist CAR-T-induced killing effects (Fig. 19).

. It can be seen from the above experiments that IFN-γ is crucial for CAR-T to kill tumor cells. IFN-γ enhances the killing ability of the CAR-T by acting on the tumor cells. In other words, priming with IFN-γ enhances the sensitivity of the tumor cells to CAR-T cells.

. Then, in the experiment of the present invention, the experimental team of the present invention establishes a peritoneal ovarian cancer model in NSG mice. The IFN-γ is administered twice before CAR-T cells are intraperitoneally injected (Fig. 16). Bioluminescence imaging show that the IFN-γ has no significant inhibitory effect on tumor growth, and CAR-T applied alone can only delay the tumor growth, whereas the combination of IFN-γ priming and CAR-T cells can lead to sustainable elimination of tumors in three of five mice. And the tumors of the remaining two mice are also significantly smaller than those in the mice of control group (Fig. 17). However, when the IFN-γ signaling pathway in the tumor cells is blocked (for example, by knocking out IFN-γR2 in the experiments in Fig. 17), the tumors are more resistant the combinational therapy of CAR-T cells combined with IFN-γ priming (Fig. 17).

. In addition, the experiments of the present invention show that the IFN-γ not only has an important effect on the CAR-T-induced killing of tumor cells, but also can enhance the killing effect of ordinary T cells (i.e., non-genetically engineered T cell) on the tumor cells (Fig. 21).

. To sum up, the in vitro and in vivo experimental results of the present invention show that the killing effect of T cells, whether is genetically engineered or is ordinary, will be enhanced when the T cells encounter the IFN-γ-primed tumor cells (that is, the IFN-γ signaling pathway is activated). This suggests that IFN-γ can enhance the sensitivity of the tumor cells to T cell-induced killing (both ordinary T cells and genetically engineered T cells (e.g., various types of CAR-T cells and/or TCR-T cells)).

. In addition to enhancing the sensitivity of tumor cells to T cells by priming tumor cells with IFN-γ, the present invention also proposes a technical solution that genetically engineering T cells (i.e., CAR-T cells) to enable the engineered T cells to secrete IFN-γ to sensitize tumor cells, thereby enhancing the effect of T cell-induced killing of tumor cells. A more optimized and more simplified therapy solution than priming tumor cells with IFN-γ is provided.

### . Embodiment 2 of B invention: a novel CAR expression vector

. Embodiment 2 of B invention provides a novel CAR expression vector, comprising nucleic acid encoding a chimeric antigen receptor (CAR) and nucleic acid encoding a tumor cell sensitizing factor, wherein the nucleic acid encoding a tumor cell sensitizing factor is: full-length or fragment of nucleic acid encoding wild-type or mutant IFN-γ.

### . Vector construction

. A plasmid vector containing an expression cassette for a chimeric antigen receptor capable of specifically secreting INF-γ is constructed. Fig. 22a is a reference diagram of structure and positional relationship of each element on an expression cassette. And Fig. 22b is a reference diagram of a plasmid vector skeleton. The specific steps are as follows: a lentiviral vector pWPXLd contains a first expression cassette of a chimeric antigen receptor targeting HER2-positive cancer cells. The first expression cassette (HER2-CAR) comprises CD8a signal peptide, a HER2 single-chain antibody variable region, a CD8 hinge region, a CD8a transmembrane domain, a 4-1BB intracellular costimulatory element and a CD3ζ intracellular domain. The second expression cassette is designed to amplify a specific fragment of human IFN-γ from T-cell cDNA with primers. The second and first IFN-γ expression cassettes are co-expressed in the lentiviral vector pWPXLd by P2A sequence (i.e., self-cleaving sequence). The whole recombinant plasmid is named as "HER2-CAR-IFN-y plasmid".

. A plasmid vector targeting HER2 is constructed in the embodiment, but the method provided by the present invention is not limited to this. The directions provided by the present invention can also construct plasmid vectors targeting common targets, including but not limited to HER2, Mesothelin, GPC-3, EGFRvIII, MUC1, CD19, CD30, BCMA, EGFR, CD133, PSCA, GD2, LewisY, etc.

. The directions provided by the present invention can also construct recombinant plasmids with similar functions using other molecular biology tools, for example, using other promoters and/or using T2A or IRES to link the first and second expression cassettes.

. Constructing CAR-T cells expressing a chimeric antigen receptor and IFN-γ

. Peripheral blood mononuclear cells (PBMCs) are isolated using lymphocyte isolation solution, and after being activated using anti-CD3 and anti-CD28 magnetic beads, the activated T cell are infected by adding the virus according to the set MOI value. The two CAR-T cells obtained are respectively named as HER2-CART and HER2-IFNG-CART cells. The expression level of the CAR in T cells is detected by flow cytometry.

### . Embodiment 3 of B invention

### . Detection of killing function and cytokine secretion of CAR-T

(1) A target cell SK-OV-3 expressing HER2 positive is seeded at wells of a 96-well plate at 1×10⁴ cells, and a MOCK group (MOCK refers to T cells that do not express the CAR construct), a HER2-CAR group, a HER2-IFNG-CAR group, a maximum release group and a spontaneous release group are set. After cell adherence, the CAR-T cells with corresponding LDH (lactate dehydrogenase) are added and co-incubated for 24h. The level of CAR-T-induced killing is detected by using a lactate dehydrogenase (LDH) kit. The cell supernatant is cryopreserved at -80°C for cytokine detection.
(2) Cytokine detection

. The expression of IFN-γ is detected by an ELISA kit from Biolegend, and the specific operations are conducted according to the instructions.

. Fig. 23 and Fig. 24 summarize detection results of killing functions and cytokine secretion of CAR-T. It can be seen from Fig. 23 that the efficacy of CAR-T cells expressing the CAR expression vector (comprising nucleic acid of IFN-γ) of the present invention in killing tumor cells is significantly enhanced, compared with the CAR-T cells only expressing HER2-CAR. It can be seen from Fig. 24 that the ability of the CAR-T cells expressing the CAR expression vector (comprising nucleic acid of IFN-γ) of the present invention to secrete the cytokine IFN-γ is also obviously enhanced, compared with the CAR-T cells only expressing HER2-CAR.

. The above experiments of the present invention show that T cells can secrete more IFN-γ by genetic engineering, which can change the microenvironment of the tumor cells and increase the sensitivity of the tumor cells to T cell immunotherapy. In addition to the CAR expression vector constructed by the present invention and the CAR-T cells expressing the vector, the efficacy of natural T cells with naturally high expression of IFN-γ or T cells (such as TCR-T cells) genetically engineered to have high expression of IFN-γ using other genetic engineering methods in killing the tumors will be stronger.

. The embodiments of the present invention are described above with reference to the accompanying drawings, but the present invention is not limited to the aforementioned specific embodiments. The aforementioned embodiments are merely illustrative and not limiting. For those of ordinary skill in the art, many forms can be made under the teaching of present invention without departing from the spirit of the present invention and the scope of the claims, all of which shall fall within the protection scope of the present invention.

## Claims

1. Use of IFN-γ in preparation of an anti-tumor adjuvant drug, the anti-tumor adjuvant drug is used for assisting treatment of a T cell preparation, wherein the IFN-γ enhances killing effect of the T cell preparation on tumor cell by sensitizing the tumor cell; the T cell preparation comprises non-genetically engineered T cell and/or genetically engineered T cell; and the IFN-γ comprises full-length or fragment of wild-type or mutant IFN-γ.

2. The use of claim 1, wherein the genetically engineered T cell comprises CAR-T cell and/or TCR-T cell.

3. The use of claim 1 or 2, wherein the IFN-γ sensitizes the tumor cell by activating an IFN-γ signaling pathway of the tumor cell.

4. The use of claim 3, wherein activation of IFN-γ signaling of the tumor cell upregulates ICAM-1 and enhances the killing effect of the T cell preparation on the tumor cell.

5. The use of any one of claims 1-4, wherein the tumor for which the anti-tumor adjuvant drug is used is solid tumor.

6. The use of any one of claims 1-4, wherein the tumor for which the anti-tumor adjuvant drug is used is ovarian tumor, breast cancer, brain gliomas, gastric cancer, colon cancer, melanoma, non-small cell lung cancer, renal cell carcinoma, bladder cancer, liver cancer or urothelial carcinoma.

7. Use of IFN-γ in preparation of an anti-tumor adjuvant drug, the anti-tumor adjuvant drug is used for assisting treatment of an immune checkpoint inhibitor, wherein the IFN-γ assists anti-tumor effect of the immune checkpoint inhibitor by sensitizing the tumor cell, and the immune checkpoint inhibitor comprises a PD-L1 inhibitor and/or a PD-1 inhibitor; and the IFN-γ comprises full-length or fragment of wild-type or mutant IFN-γ.

8. The use of claim 7, wherein the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

9. The use of claim 7 or 8, wherein the tumor for which the anti-tumor adjuvant drug is used is solid tumor.

10. The use of any one of claims 7-9, wherein the tumor for which the anti-tumor adjuvant drug is used is ovarian tumor, breast cancer, brain gliomas, gastric cancer, colon cancer, melanoma, non-small cell lung cancer, renal cell carcinoma, bladder cancer, liver cancer or urothelial carcinoma.

11. Use of IFN-γ in preparation of an anti-tumor adjuvant drug, the anti-tumor adjuvant drug is used for assisting treatment of a T cell preparation and an immune checkpoint inhibitor, wherein the IFN-γ assists anti-tumor effect of the T cell preparation and the immune checkpoint inhibitor by sensitizing the tumor cell; the immune checkpoint inhibitor comprises a PD-L1 inhibitor and/or a PD-1 inhibitor; the T cell preparation comprises non-genetically engineered T cell and/or genetically engineered T cell; and the IFN-γ comprises full-length or fragment of wild-type or mutant IFN-γ.

12. The use of claim 11, wherein the genetically engineered T cell comprises CAR-T cell and/or TCR-T cell.

13. The use of claim 11 or 12, wherein the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

14. The use of any one of claims 11-13, wherein the anti-tumor adjuvant drug stimulates the tumor cell to produce ICAM-1 and enhance killing ability of the T cell preparation on the tumor cell.

15. The use of any one of claims 11-14, wherein the tumor is solid tumor.

16. The use of any one of claims 11-14, wherein the tumor is ovarian tumor, breast cancer, brain gliomas, gastric cancer, colon cancer, melanoma, non-small cell lung cancer, renal cell carcinoma, bladder cancer, liver cancer or urothelial carcinoma.

17. A kit used for detecting anti-tumor effect of an immune checkpoint inhibitor, the immune checkpoint inhibitor comprises a PD-L1 inhibitor and/or a PD-1 inhibitor, wherein the kit comprises detecting whether there is any obstruction of IFN-γ signaling pathway of tumor cell.

18. The kit of claim 17, wherein the kit comprises detecting an expression level of ICAM-1 or IFN-γR2 in the tumor cell.

19. The kit of claim 17 or 18, wherein the PD-L1 inhibitor comprises one or more of Atezolizumab, Avelumab and Durvalumab; and the PD-1 inhibitor comprises one or more of Pembrolizumab, Nivolumab, Cemiplimab, Toripalimab, Tislelizumab, Tyvyt and Camrelizumab.

20. The kit of any one of claims 17-19, wherein the tumor is solid tumor.

21. The kit of any one of claims 17-19, wherein the tumor is ovarian tumor, breast cancer, brain gliomas, gastric cancer, colon cancer, melanoma, non-small cell lung cancer, renal cell carcinoma, bladder cancer, liver cancer or urothelial carcinoma.

22. An anti-tumor drug for assisting anti-tumor immunotherapy, wherein the anti-tumor drug comprises full-length or fragment of wild-type or mutant IFN-γ, and assists anti-tumor effect of a T cell preparation and/or an immune checkpoint inhibitor by sensitizing tumor cell.

23. The anti-tumor drug of claim 22, wherein the anti-tumor drug comprises targeting vector, and the targeting vector can transport the full-length or fragment of wild-type or mutant IFN-γ to the tumor cell.

24. The anti-tumor drug of claim 22 or 23, wherein the anti-tumor drug is used together with the T cell preparation and/or the immune checkpoint inhibitor.

25. The anti-tumor drug of any one of claims 22-24, wherein the anti-tumor drug further comprises any pharmaceutically acceptable vector and/or auxiliary.

26. A CAR expression vector, comprising nucleic acid encoding chimeric antigen receptor (CAR) and nucleic acid encoding tumor cell sensitizing factor, wherein the nucleic acid encoding tumor cell sensitizing factor is nucleic acid encoding full-length or fragment of wild-type or mutant IFN-γ.

27. The CAR expression vector of claim 26, wherein the nucleic acid encoding chimeric antigen receptor (CAR) is linked to the nucleic acid encoding tumor cell sensitizing factor by a sequence encoding self-cleaving peptide.

28. The CAR expression vector of claim 26, wherein the nucleic acid encoding chimeric antigen receptor (CAR) comprises nucleic acid encoding single-chain antibody variable region (ScFv) targeted tumor-specific antigen, CD8a signal peptide, CD8a hinge region, CD8a transmembrane domain, 4-IBB intracellular costimulatory element and CD3ζintracellular domain.

29. The CAR expression vector of claim 28, wherein the tumor-specific antigen comprises one or more of HER2, Mesothelin, GPC-3, EGFRvIII, MUC1, CD19, CD30, BCMA, EGFR, CD133, PSCA, GD2 and LewisY.

30. The CAR expression vector of claim 26, wherein the sequence encoding self-cleaving peptide is a P2A sequence.

31. The CAR expression vector of claim 26, wherein sequence of the nucleic acid encoding chimeric antigen receptor (CAR) is shown as SEQ ID NO:1; sequence of the nucleic acid encoding IFN-γ is shown as SEQ ID NO:2; and the sequence encoding self-cleaving peptide is shown as SEQ ID NO:3.

32. A lentivirus comprising the CAR expression vector of any one of claims 26-31.

33. The lentivirus of claim 32, wherein the lentivirus is pWPXLd.

34. A CAR-T cell, wherein the CAR-T cell is transfected with vector as described in (a) or (b): (a) the CAR expression vector of any one of claims 26-31; and (b) a CAR expression vector comprising nucleic acid encoding CAR and nucleic acid encoding IFN-γ.

35. An anti-tumor drug, wherein the anti-tumor drug comprises the CAR-T cell of claim 34 and pharmaceutically acceptable adjuvant and/or auxiliary.

36. The anti-tumor drug of claim 35, wherein the tumor is solid tumors.

37. The anti-tumor drug of claim 35, wherein the tumor is ovarian tumor, breast cancer, brain gliomas, gastric cancer, colon cancer, melanoma, non-small cell lung cancer, renal cell carcinoma, bladder cancer, liver cancer or urothelial carcinoma.

38. A T cell, wherein the T cell expresses CAR and IFN-γ.

39. The T cell of claim 38, wherein the T cell comprises nucleic acid encoding the CAR and nucleic acid encoding the IFN-γ.

40. An anti-tumor drug, wherein the anti-tumor drug comprises the T cell of claim 38 or 39 and pharmaceutically acceptable adjuvant and/or auxiliary.

41. The anti-tumor drug of claim 40, wherein the tumor is solid tumor.

42. The anti-tumor drug of claim 40, wherein the tumor is ovarian tumor, breast cancer, brain gliomas, gastric cancer, colon cancer, melanoma, non-small cell lung cancer, renal cell carcinoma, bladder cancer, liver cancer or urothelial carcinoma.

43. A preparation method for T cell expressing CAR and IFN-γ, wherein the preparation method uses vector to transfect nucleic acid encoding CAR and nucleic acid encoding IFN-γ into the T cell.
